(19) Europäisches Patentamt
European Patent Office
Office européen des brevets

(11) Numéro de publication: **0 411 111 B1**

(12) # FASCICULE DE BREVET EUROPEEN

(45) Date de publication de fascicule du brevet: **02.11.94**

(51) Int. Cl.[5]: **C08G 73/02**, C07C 211/63, C07C 279/12, C07C 323/25, A01N 33/12, A23L 3/34, C02F 1/50

(21) Numéro de dépôt: **90904197.2**

(22) Date de dépôt: **15.02.90**

(86) Numéro de dépôt internationale : **PCT/BE90/00009**

(87) Numéro de publication internationale : **WO 90/09405 (23.08.90 90/20)**

Le dossier contient des informations techniques présentées postérieurement au dépôt de la demande et ne figurant pas dans le présent fascicule.

(54) **COMPOSE D'AMMONIUM, COMPOSITION LE CONTENANT ET PROCEDE DE DESINFECTION.**

(30) Priorité: **15.02.89 BE 8900149**

(43) Date de publication de la demande: **06.02.91 Bulletin 91/06**

(45) Mention de la délivrance du brevet: **02.11.94 Bulletin 94/44**

(84) Etats contractants désignés: **AT BE CH DE DK ES FR GB IT LI LU NL SE**

(56) Documents cités:
**WO-A-87/02221**
**US-A- 3 931 319**
**US-A- 4 217 914**

(73) Titulaire: **FABRICOM AIR CONDITIONING S.A.**
**rue du Montenégro, 138 à 144**
**B-1060 Bruxelles (BE)**

(72) Inventeur: **LEGROS, Alain**
**11, rue Docteur-Maître**
**B-6290 Nalinnes (BE)**

(74) Mandataire: **De Palmenaer, Roger et al**
**Bureau Vander Haeghen S.A.**
**Rue Colonel Bourg 108 A**
**B-1040 Bruxelles (BE)**

EP 0 411 111 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à un composé d'ammonium quaternaire répondant à la formule suivante :

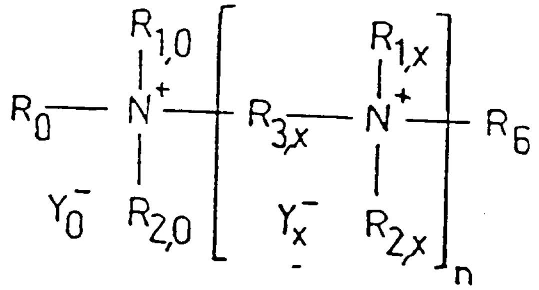

(1)

dans laquelle :

n est un nombre entier supérieur ou égal à 1;

x est un nombre compris entre 1 et n ;

$R_o$ et $R_6$ qui peuvent être identiques ou différents désignent un radical hydrocarboné éventuellement insaturé, ce radical contenant de 1 à 22 atomes de carbone;

$R_{1,y}$, $R_{2,y}$ pour y compris entre 0 et n désignent un groupe hydrocarboné éventuellement ramifié, insaturé et/ou substitué par un ou plusieurs halogènes ou par un groupement hydrocarboné, $R_{1,y}$ et $R_{2,y}$ pouvant contenir jusqu'à 22 atomes de carbone; au moins un radical $R_o$, $R_6$, $R_{1,y}$ ou $R_{2,y}$ désigne un radical contenant de 10 à 22 atomes de carbone ;

$R_{3,x}$, pour x compris entre 1 et n, désigne un groupe hydrocarboné éventuellement insaturé et/ou ramifié pouvant contenir jusqu'à 10 atomes de carbone, ou un groupe de formule :

$- (CH_2)_s - S - (CH_2)_t-$
$- (CH_2)_s - S - S - (CH_2)_t-$
$- (CH_2)_s - S\ 0 - (CH_2)_t-$

où s et t sont des nombres entiers,

ces groupes étant éventuellement substitués par des groupements hydrocarbonés,

$R_{3,x}$ pouvant être identique ou différent de $R_{3,x+1}$, et

$Y_o^-$ et $Y_x^-$ désignent un anion, de préférence, un atome d'halogène, le poids moléculaire de ce composé étant compris entre 1000 et 2654,

ou un composé répondant à l'une quelconque des formules suivantes :

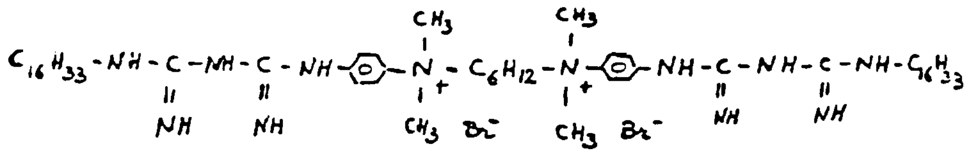

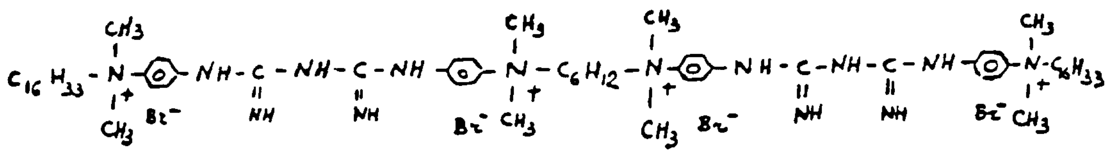

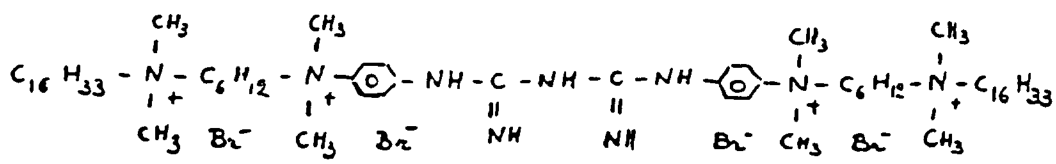

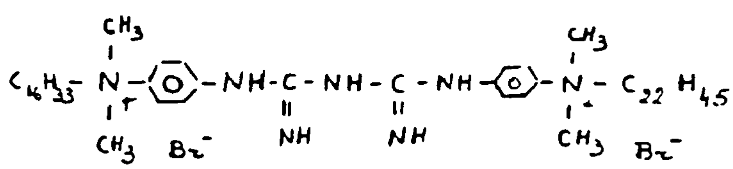

Elle a encore pour objet :

- une composition destinée à la désinfection de liquides et/ou de surfaces ou à la conservation d'aliments ou de boissons , cette composition contenant au moins un composé d'ammonium suivant l'invention, et
- un procédé de désinfection.

On connaît par le document US 3,931,319 des polymères d'ammonium quaternaire obtenus par condensation d'une amine tertiaire avec un excès de 1,4-dihalo-2-butène et qui présentent en fin de chaîne des groupes alkyle primaires ou secondaires contenant de 1 à 20 atomes de carbone, tels que des groupes hexadécyle, ces polymères présentant des propriétés microbicides contre des bactéries Aerobacter aerogenas et Pseudomonas aeruginosa.

On connaît également des compositions désinfectantes contenant un composé d'ammonium quaternaire et un ion de cuivre. Ces compositions connues ont un poids moléculaire important et ne permettent pas une destruction rapide et efficace de bactéries telles que l'Acinetobacter Calcoaceticus Iwoffi ou encore l'Acetobacter hansenii.

La présente invention vise à remédier à ces inconvénients.

Elle a entre autres pour objet une composition désinfectante qui permet de détruire rapidement et de façon efficace des bactéries difficiles a détruire telles que l'Acetobacter hansenii et le Pseudomonas stutzeri.

Elle a également pour objet une composition ne présentant pas les problèmes de dégradations dues à la température ou à une oxydation.

Elle a encore pour objet une composition ne présentant aucun problème d'odeur.

Description de l'invention

La présente invention est relative à un composé d'ammonium quaternaire répondant à la formule suivante :

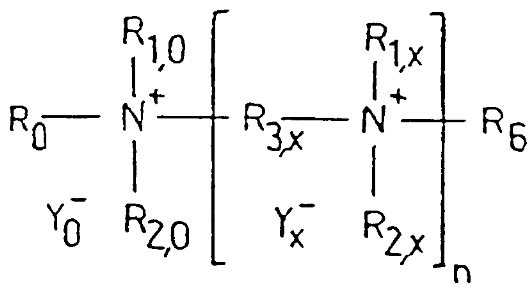

(1)

dans laquelle :
n est un nombre entier supérieur ou égal à 1;
x est un nombre compris entre 1 et n :
$R_o$ et $R_6$ qui peuvent être identiques ou différents désignent un radical hydrocarboné éventuellement insaturé, ce radical contenant de 1 à 22 atomes de carbone;
$R_{1,y}$, $R_{2,y}$ pour y compris entre 0 et n désignent un groupe hydrocarboné éventuellement ramifié, insaturé et/ou substitué par un ou plusieurs halogènes ou par un ou plusieurs groupements hydrocarbonés
$R_{1,y}$ et $R_{2,y}$ pouvant contenir jusqu'à 22 atomes de carbone,
$R_{3,x}$ pour x compris entre 1 et n, désigne un groupe hydrocarboné éventuellement insaturé et/ou ramifié pouvant contenir jusqu'à 10 atomes de carbone, ou un groupe de formule :

$-(CH_2)_s-S-(CH_2)_t-$
$-(CH_2)_s-S-S-(CH_2)_t-$
$-(CH_2)_s-SO-(CH_2)_t-$,

où s et t sont des nombres entiers,
ces groupes étant éventuellement substitués par des groupements hydrocarbonés,
$R_{3,x}$ pouvant être identiques ou différents de $R_{3,x+1}$ ;
$Y_o^-$ et $Y_x^-$ désignent un anion, de préférence, un atome d'halogène.
le poids moléculaire de ce composé étant compris entre 1000 et 2654
ou un composé répondant à l'une quelconque des formules suivantes :

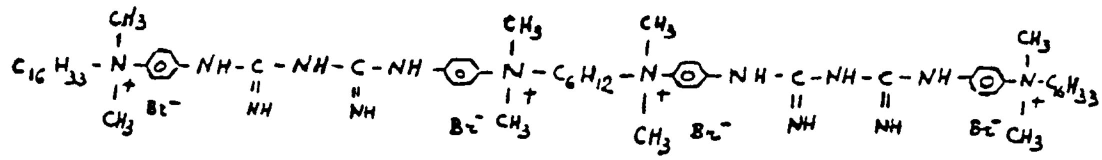

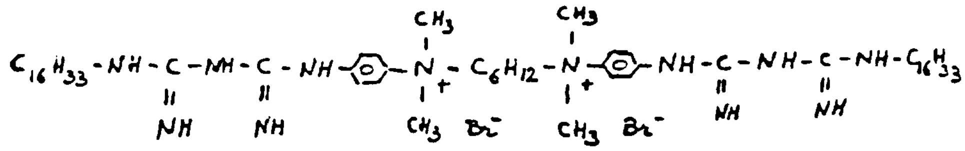

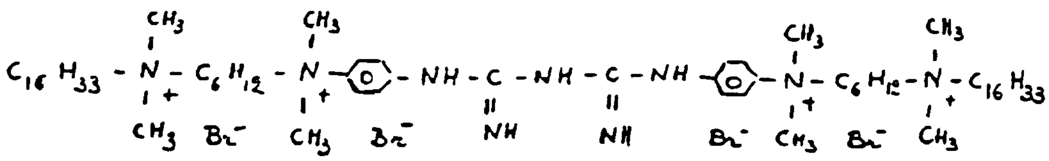

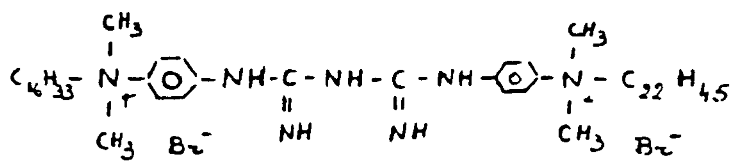

De préférence, au moins un radical $R_o$, $R_6$, $R_{1,y}$ ou $R_{2,y}$ du composé suivant l'invention contient de 10 à 22 et en particulier de 12 à 16 atomes de carbone.

Avantageusement, pour au moins un x compris entre 1 et n, $R_{3,x}$ désigne un groupe

$-(CH_2)_w-$ avec w compris entre 1 et 10 ;

ou

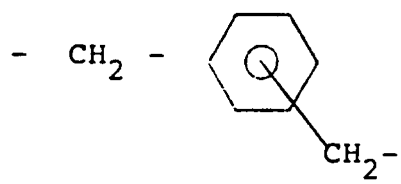 ,

un des substituants $-CH_2-$ pouvant être en position ortho, méta ou para ; ou

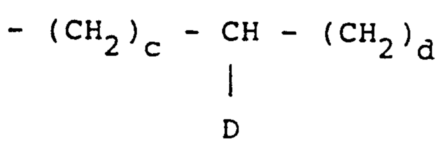

dans laquelle D désigne un atome d'hydrogène ou un radical $C_{1-4}$ alkyl,

c et d sont des nombres entiers inférieurs à 5 dont l'un peut avoir une valeur égale à 0, tandis que la somme c + d est au moins égale à 1 et au maximum égale à 8,

le radical pouvant contenir au maximum deux doubles liaisons ; ou

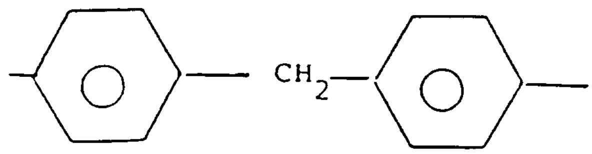

La présente invention a encore pour objet une composition destinée à la désinfection de liquides et/ou surfaces ou à la conservation d'aliments ou boissons, cette composition contenant un composé de formule 1 suivant l'invention.

Selon une caractéristique de la composition suivant l'invention, elle contient au moins un ion d'un métal choisi parmi le fer, le cuivre, l'argent ou un mélange de tels ions.

Selon une autre caractéristique de la composition suivant l'invention, elle contient également un composé d'ammonium quaternaire de formule générale

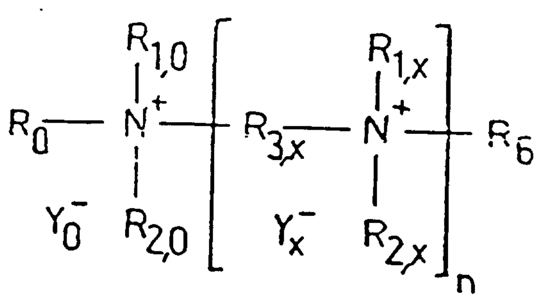

(2)

dans laquelle n, x, $y_o^-$, $y_x^-$, $R_o$, $R_6$, $R_{1,x}$ et $R_{2,x}$ pour x compris entre O et n ont les significations données ci-dessus et dans laquelle $R_{3,x}$ désigne un groupe hydrocarboné éventuellement insaturé et/ou ramifié pouvant contenir jusqu'a 10 atomes de carbones, ou
un groupe de formule :

$-(CH_2)_s-S-(CH_2)_t$
$-(CH_2)_s-O-(CH_2)_t$
$-(CH_2)_s-S-S-(CH_2)_t$
$-(CH_2)_s-SO-(CH_2)_t$

où s et t sont des nombres entiers,
ces groupes étant éventuellement substitués,
le poids moléculaire de ce composé étant inférieur à 5000, et, de préférence, compris entre 1000 et 4500.

De préférence, $R_o$ et/ou $R_6$ et/ou $R_{1,y}$ et/ou $R_{2,y}$ du deuxième composé désignent un radical contenant de 10 à 22 atomes de carbone.

$R_o$ et $R_6$ désignent avantageusement un groupe contenant 16 atomes de carbone.

Selon une particularité de la composition suivant l'invention, s et t sont des nombres entiers compris entre 1 et 3.

Selon une autre particularité de la composition suivant l'invention, $R_{3,x}$ du deuxième composé pour au moinx un x compris entre 1 et n, désigne un groupe :

$-(CH_2)_w$ avec w compris entre 1 et 10

ou

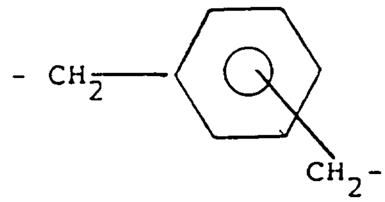

un des substituants $-CH_2$ - pouvant être en position ortho, méta ou para
ou

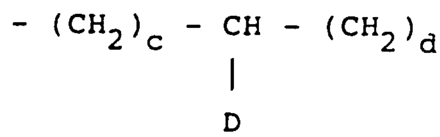

dans laquelle D désigne un atome d'hydrogène ou un radical $C_{1-4}$ alkyl,
c et d sont des nombres entiers inférieurs à 5 dont l'un peut avoir une valeur égale à 0, tandis que la somme c + d est au moins égale à 1 et au maximum égale à 8,
le radical pouvant contenir au maximum deux doubles liaisons ;
ou

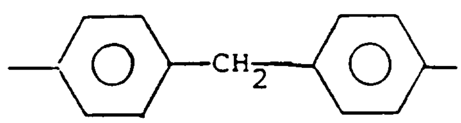
;

quant à $R_{1,v}$ et $R_{2,v}$, pour au moins un v compris entre 0 et n, ils désignent un radical alkyl inférieur, de préférence $-CH_3$.

Dans une forme de réalisation particulière de la composition suivant l'invention, le rapport en poids du premier composé de formule 1 ayant un poids moléculaire compris entre 1000 et 2654 et du composé de formule 2 ayant un poids moléculaire inférieur à 5000 est compris entre 0,1 et 10, de préférence entre 0,5 et 6.

Dans une autre forme, le composé de formule 1 a un poids moléculaire compris entre 1000 et 2654 et le composé de formule 2 a un poids moleculaire compris entre 1000 et 4500, le rapport en poids de ces composés étant d'environ 1.

La présente invention a également pour objet un procédé de désinfection dans lequel on ajoute au moins une composition suivant l'invention ou dans lequel on met en contact une surface à désinfecter avec au moins une composition suivant l'invention.

Elle a encore pour objet un procédé de conservation de boissons ou d'aliments dans lequel on ajoute auxdites boissons une composition suivant l'invention ou dans lequel on injecte dans lesdits aliments une composition suivant l'invention ou dans lequel on trempe lesdits aliments dans une composition suivant l'invention.

D'autres particularités et détails de l'invention ressortiront de la description détaillée suivante d'exemples de composition.

## EXEMPLES DE SYNTHESE

### Exemple 1 (Comparatif)

On a mélangé 6,58 g de N,N,N',N' tétraméthylisopropanol diamine, avec 5,63 g de 1,2 dibromoéthane et 30,9 ml d'eau.

On a porté le mélange à 60° C et on a agité ledit mélange à cette température pendant 12 heures.

On a ensuite porté le mélange à 85° C et on y a ajouté 9,16 g de bromohexadecane.

On a agité la solution pendant 24 heures.

On a ensuite dilué la solution obtenue avec une quantité d'eau correspondant à 20 fois le poids de la solution et on a traité ladite solution au charbon actif.

La composition ainsi obtenue n'est pas une composition suivant l'invention puisque $R_{3,x}$ n'est pas un groupe hydrocarboné éventuellement substitué par un groupement hydrocarboné.

Cette composition présentait une faible solubilité dans l'eau.

Le composé d'ammonium avait un poids moléculaire de 1425.

Exemple 2

De façon similaire à l'exemple 1, on a préparé une composition suivant l'invention à partir de 5,86 g de N,N,N',N' tétraméthylpropane diamine, 5,63 g de 1,2-dibromoéthane et 9,16 g de 1-bromo hexadecane. Le composé d'ammonium avait un poids moléculaire de 1370.

La composition obtenue, après dilution, avait une coloration blanchâtre et contenait 7,25 % de matière sèche.

Exemple 3

En utilisant le mode opératoire décrit dans l'exemple 1, on a préparé une composition suivant l'invention à partir de 3,91 g de N,N, N',N' tétraméthylpropane diamine, 2,82 g de 1,2-dibromoéthane, 34,5 ml d'eau et 9,16 g de 1 - bromohexadecane. Le composé d'ammonium quaternaire avait un poids moléculaire de 1059.

Après dilution de la composition et le traitement de celle-ci au charbon actif, la composition contenait 6,39 % de matière sèche.

Exemple 4 (Comparatif)

On a opéré comme dans l'exemple 3 si ce n'est qu'on a utilisé 4,39 g de N, N, N', N' tétraméthyle isopropanol diamine à la place des 3,91 g de N, N, N', N' tétraméthylpropane diamine.

La composition a été purifiée au charbon actif et à la concentration après dilution de la composition était de 6,34 %.

Le poids moléculaire du composé d'ammonium quaternaire était d'environ 1091.

Exemple 5

On a agité 14,77 g de N,N,N',N' tétraméthylhexane diamine (0,086 mole) et 11,54 g de 1,3-dibromopropane (0,057 mole) dans 60 ml d'eau pendant 3 heures à une température de 60° C.

On a porté ensuite le mélange à 90° C et on a ajouté à ce mélange 17,45 g (0,057 mole) de 1 - bromohexadecane. Ce nouveau mélange a été maintenu sous agitation pendant 24 heures.

Le rendement de la réaction était supérieur à 90 %.

Le poids moléculaire du composé d'ammonium quaternaire était de 1531.

Exemple 6 (Comparatif)

On a agité 17,92 g de N,N,N',N' tétraméthylhexane diamine (0,1 mole) et 18,37 g de 1,3 dibromopropane (0,09 mole) pendant 2 heures dans 60 ml d'eau à 60° C.

On a ensuite porté le mélange à une température de 90° C et on a ajouté 7,94 g de 1-bromohexadecane (0,026 mole). On a maintenu le mélange sous agitation pendant 24 heures.

La solution aqueuse a été purifiée par passage dans une colonne de charbon actif.

Le poids moléculaire du composé d'ammonium quaternaire était de 3402.

Exemple 7 (Comparatif)

On a préparé un polymère d'ammonium quaternaire comme dans l'exemple 6. Toutefois les 0,026 moles de 1-bromohexadecane ont été remplacées par 0,026 mole de 1-bromodecane.

La composition obtenue était moussante et légèrement jaune. Le rendement de la réaction était de 96 %.

Le polymère d'ammonium avait un poids moléculaire de 3234.

Exemple 8

On a mélangé dans 73,35 g d'eau 77,54 g de N,N,N',N'-tétraméthylhexane diamine et 60,57 g de 1,3-dibromopropane. Ce mélange a été maintenu sous agitation à température ambiante. Une réaction exothermique a été observée.

Après 12 heures de réaction, on a ajouté 50 ml d'eau et on a porté le mélange à 60°C pendant 3 heures. On a obtenu ainsi une composition contenant environ 200 g de polymère hydrosoluble.

Exemple 9

On a ajouté à 28,3 g de la solution du polymère de l'exemple 8, 6,64 g de bromodécane et 15,5 ml d'eau. On a porté la température du mélange à 90°C pendant 24 heures.

La composition obtenue contenait un polymère hydrosoluble ayant un poids moléculaire d'environ 1363.

Exemple 10 (Comparatif)

On a mélangé à 85°C 17,23 g de N,N,N',N'-tétraméthylhexane diamine, 61,07 g de 1-bromohexadécane et 70 ml d'eau. Ce mélange a été maintenu sous agitation pendant 24 heures. La composition obtenue qui contenait un composé d'ammonium quaternaire a été purifiée sur du charbon actif.

Le rendement de la réaction était supérieur à 90 %.

Le composé d'ammonium quaternaire obtenu dans cet exemple est un produit présentant une activité antibactérienne.

Exemple 11 (Comparatif)

On a préparé un polymère d'ammonium de la manière décrite dans l'exemple 6, mais on a remplacé le 1-bromohexadécane (0,026 mole) par du 1-bromododécane (0,026 mole).

Le produit final a été purifié sur charbon actif.

Le rendement global de la réaction était de 96 %.

Le poids moléculaire du polymère préparé était d'environ 3290.

Exemple 12.

On a ajouté à 28,3 g de la solution aqueuse de polymère de l'exemple 8, 7,48 g de 1-bromododécane et 14,2 ml d'eau.

On a maintenu le mélange sous agitation et a une température de 90°C pendant 24 heures. Après purification par passage sur charbon actif et après dilution, on a obtenu une composition contenant 9,87 % de matière sèche. Le poids moléculaire du composé obtenu était de 1419.

Exemple 13 (Comparatif)

172,3g*de N,N,N',N'- tétraméthylhexane diamine et 201,9 g*de 1,3-dibromopropane ont été mis en réaction pendant 182 heures à 25° C dans 550 ml d'un mélange* contenant 80 % de diméthylformamide et 20 % d'eau. Après cette réaction, on a précipité le polymère à l'aide d'acétone anhydre. Le rendement de la réaction a été de 80 % et le polymère contenait 41,37 % de bromure.

Le poids moléculaire de ce polymère était de 8412.

Exemple 14

On a préparé un réactif intermédiaire en mélangeant en quantité stoechiométrique du tribomure de phosphore et du 2,2' dithiodiéthanol dans un milieu éther. On a chauffé ce mélange a reflux (40° C) et sous agitation pendant 18 heures. Le mélange de réaction a été lavé deux fois et on a évaporé l'éther. On a récupéré ainsi 3,5 g de réactif intermédiaire.

(* soit une solution 2 molaire pour chaque réactif ).

On a ensuite ajouté à de l'eau 4,31 g de N,N,N',N' tétraméthylhexane diamine et 3,5 g du réactif intermédiaire et on a porté ce mélange a 60° C (chauffage à reflux et sous agitation) pendant 24 heures. Ensuite on a ajouté 7,64 g de bromohexadécane et on a porté le mélange à 90°C pendant 24 heures (chauffage a reflux et sous agitation).

Le mélange ainsi obtenu était limpide, incolore et visqueux.

Le composé d'ammonium obtenu avait un poids moléculaire de 1235, et répondait à la formule :

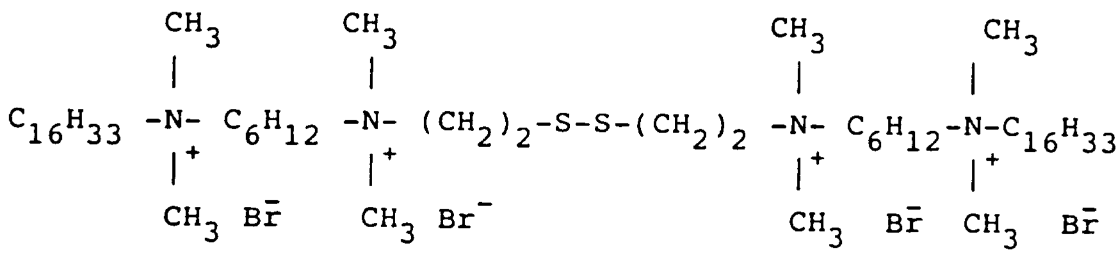

Exemples 15 à 29

On a préparé divers composés en mélangeant les réactifs suivants :

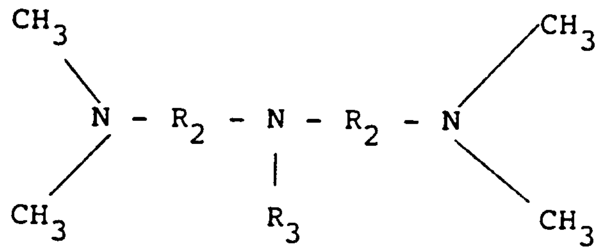

$R_1$- X

On a porté ce mélange à une température comprise entre 30 et 100° C pendant 24 heures (à reflux). Ensuite on a ajouté audit mélange un réactif $R_4$-X et on a chauffé à reflux le mélange à une température comprise entre 50° C et 120° C pendant 24 heures.

Le tableau suivant donne les produits ou composés que l'on a préparés, ces produits ayant la formule :

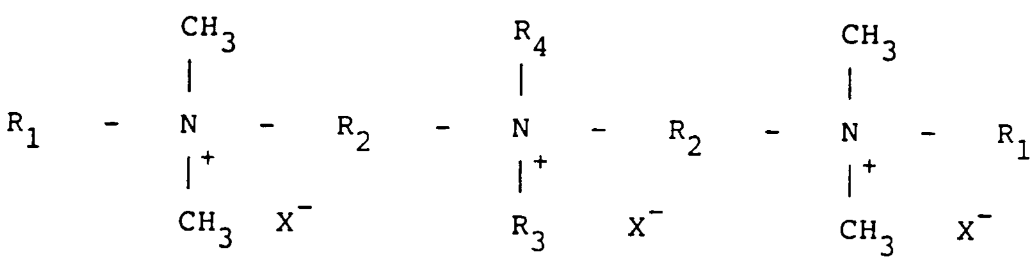

## TABLEAU

X = Br

| Exemple | $R_1$ | $R_2$ | $R_3$ | $R_4$ | Poids moléculaire |
|---|---|---|---|---|---|
| 15 | $C_{16}H_{33}$ | $C_3H_6$ | $C_{22}H_{45}$ | $CH_3$ | 1201 |
| 16 | $C_{16}H_{33}$ | $C_3H_6$ | $-CH_2-$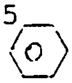 | $CH_3$ | 983 |
| 17 | $C_{18}H_{37}$ | $C_3H_6$ | $C_{16}H_{33}$ | $CH_3$ | 1174 |
| 18 | $C_{18}H_{37}$ | $C_3H_6$ | $C_{18}H_{37}$ | $CH_3$ | 1201 |
| 19 | $C_{18}H_{37}$ | $C_3H_6$ | $C_{22}H_{45}$ | $CH_3$ | 1258 |
| 20 | $C_{16}H_{33}$ | $C_6H_{12}$ | $C_{16}H_{33}$ | $CH_3$ | 1202 |
| 21 | $C_{16}H_{33}$ | $C_6H_{12}$ | $C_{18}H_{37}$ | $CH_3$ | 1230 |
| 22 | $C_{16}H_{33}$ | $C_6H_{12}$ | $C_{22}H_{45}$ | $CH_3$ | 1286 |
| 23 | $C_{16}H_{33}$ | $C_6H_{12}$ | $-CH_2-$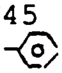 | $CH_3$ | 997 |
| 24 | $C_{16}H_{33}$ | $C_6H_{12}$ | $-CH_2-$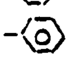 | $C_{16}H_{33}$ | 1222 |
| 25 | $C_{18}H_{37}$ | $C_6H_{12}$ | $C_{16}H_{33}$ | $CH_3$ | 1257 |
| 26 | $C_{18}H_{37}$ | $C_6H_{12}$ | $C_{18}H_{37}$ | $CH_3$ | 1286 |
| 27 | $C_{18}H_{37}$ | $C_6H_{12}$ | $C_{22}H_{45}$ | $CH_3$ | 1342 |
| 28 | $C_{18}H_{37}$ | $C_6H_{12}$ | $-CH_2-$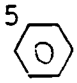 | $CH_3$ | 1053 |
| 29 | $C_{22}H_{45}$ | $C_6H_{12}$ | $C_{22}H_{45}$ | $CH_3$ | 1454 |

Exemple 30

On a fait réagir en milieu aqueux 2,23 g de dicyanamide de sodium et 10,95 g de p-iodoaniline à 120° C pendant 24 heures. On a alors ajouté au mélange 2 ml d'HCl concentré et ensuite 8,62 g de tétraméthyl diamine hexane. Après avoir chauffé ce mélange a 50° C pendant 24 heures, on a ajouté 15, 27 g de bromohexadécane et on a porté le mélange a 90° C pendant 24 heures.

Après décantation, on a éliminé le surnageant aqueux et on a lavé à l'eau le produit décanté.

Ce produit répond ait à la formule suivante :

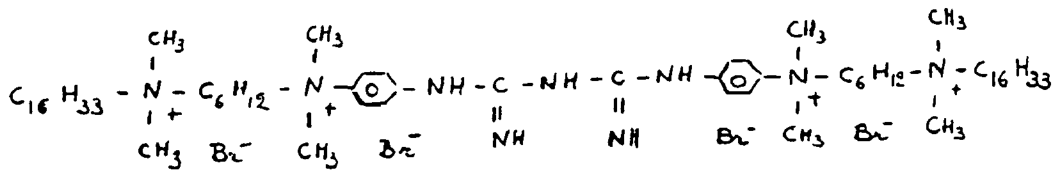

et avait un poids moléculaire de 1460

Exemple 31

On a fait réagir sous atmosphère d'argon 2,23 g de dicyanamide de sodium et 6,04 g de bromohexane à 140 ° C pendant 10 heures.

Après avoir ajouté au mélange 4,3 g de p-iodoaniline, on a maintenu le mélange à 240° C pendant 15 heures. On a ensuite ajouté 2 ml d'HCl concentré, de l'eau et 2,15 g de tétraméthyl 1,6 diamine hexane avant de porter le mélange à 90° C pendant 10 heures.

On a ainsi récupéré par décantation un produit qui après lavage à l'eau répondait à la formule

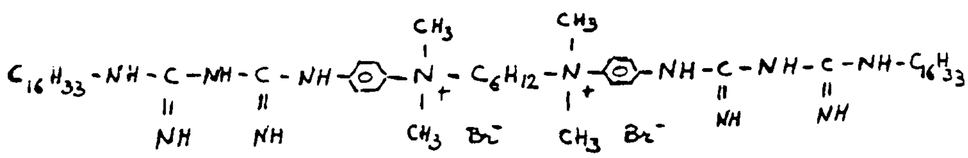

(poids moléculaire : 1133).

Exemple 32

On a fait réagir en milieu aqueux sous atmosphère d'azote 2,23 g de dicyanamide de sodium avec 8,6 g de p-iodoaniline à 140°C pendant 10 heures.

Après avoir ajouté 2 ml HCl concentré de l'eau et 2,15 g de tétraméthyl diamine hexane, on a maintenu la température du mélange à 70° C pendant 15 heures. Ensuite on a ajouté au mélange 6,74 g de bromohexadecane et on a porté la température du mélange à 90° C, tempérautre que l'on a maintenue pendant 10 heures.

On a récupéré du milieu un composé que l'on a lavé avec de l'eau.

Le composé répondait à la formule suivante (poids moléculaire 1553) :

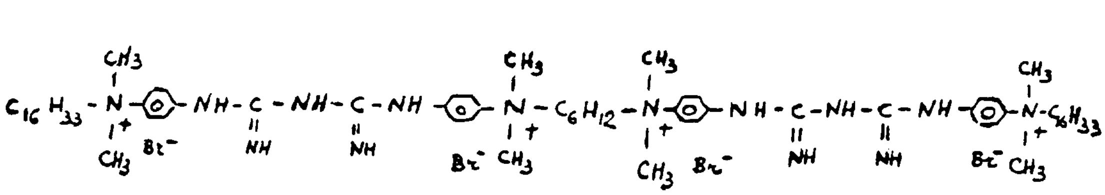

Exemple 33

On a fait réagir à 110° C pendant 24 heures du 1-bromo docosane avec un excès de diméthylamine dans un milieu aqueux.

On a ensuite bouilli ce mélange dans un milieu basique et par extraction à l'éther et distillation on a récupéré un réactif intermédiaire.

On a fait réagir 2,23 g de cyanamide de sodium avec 8,6 g de p-iodioaniline à 140° C pendant 10 heures et sous atmosphère d'argon.

On a alors ajouté 2 ml d'HCl concentré, de l'eau et 6,74 g d'hexadécyl diméthylamine.

On a porté la température du mélange à 70° C pendant 15 heures et on a ajouté 8,84 g de réactif intermédiaire.

Le mélange a alors été porté à 90° C pendant 10 heures.

On a récupéré ainsi un produit de formule :

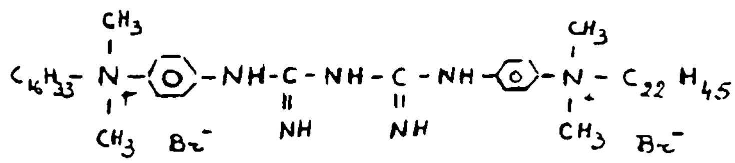

(poids moléculaire 1034).

EXEMPLE D'UTILISATION

Pour effectuer ces essais, on a utilisé la méthode bien connue de dilutions successives dans des tubes à essais et ensemencement sur un milieu de culture tryptone-glucose-extract agar et en utilisant un polyphosphate comme neutralisant.

Les essais de désinfection d'eau dont il est question ci-dessous ont été effectués dans de l'eau de ville ayant une dureté d'environ 35° F, préalablement filtrée sur un filtre millipore de 0,22µ , après trois repiquages successifs de chacune des souches.

L'action inattendue résultant de l'utilisation d'au moins un composé d'ammonium de formule 1 de préférence en association avec des ions de cuivre et/ou d'argent est illustrée dans les exemples suivants dans lesquels la vitesse de destruction de divers micro-organismes tels que ceux infestant les eaux de piscines de natation.

Dans le cas où la composition suivant l'invention est utilisée pour la désinfection d'eau de piscines, on utilise a raison de 0,5 à 10 ppm (parties par million) de composé d'ammonium par rapport au poids du milieu aqueux a désinfecter et à raison de 0,5 à 5 ppm de cuivre et de 1 à 50 ppb (parties par milliard) d'argent.

Exemple I

On a traité une eau contenant 490.000 germes/ml de Streptocoque faecalis ATCC 6569 par diverses compositions. Les résultats des traitements sont donnés dans le tableau I suivant :

TABLEAU I

| Quantité et nature des ingrédients ajoutés | temps de contact minutes | % de bactéries restantes |
|---|---|---|
| - 0,5 ppm du composé de l'exemple 10 (comparatif) + 3 ppm du composé de l'exemple 6 (comparatif) | 5 | 2,45 |
| - idem + 1 ppm Cu + 5 ppb Ag | 5 | 0,84 |
| - 1 ppm Cu + 5 ppb Ag | 5 | ± 100 |
| - 1,5 ppm du composé de l'exemple 1 (comparatif) + 1,5 ppm du composé de l'exemple 2 (invention) | 5 | 0,39 |
| - idem + 1 ppm Cu + 5 ppb Ag | 5 | 0,0006 |

Cet exemple montre que l'utilisation d'un composé d'ammonium quaternaire suivant l'invention ayant un poids moléculaire compris entre 1000 et 2654 en mélange avec un autre composé d'ammonium quaternaire ayant un poids moléculaire compris entre 1000 et 4500 avec du cuivre et de l'argent permet une destruction plus rapide du Streptocoque faecalis par rapport à celle obtenue en utilisant un composé d'ammonium quaternaire non selon l'invention ayant un poids moléculaire compris entre 1000 et 4500 en

mélange avec un autre composé d'ammonium quaternaire ayant un poids moléculaire inférieur à 1000, avec du cuivre et de l'argent.

Exemple II

On a traité une eau contenant 620.000 germes/ml d'Escherichia coli ATCC 11229 par diverses compositions. Le tableau II suivant reprend les résultats de ces traitements.

TABLEAU II

| Quantité et nature des ingrédients ajoutés | temps de contact minutes | % de bactéries restantes |
|---|---|---|
| -0,5 ppm du composé de l'exemple 10 (comparatif) + 3 ppm du composé de l'exemple 2 (invention) | 5 | 1,22 |
| -idem + 1 ppm Cu + 5 ppb Ag | 5 | 0,1 |
| -1 ppm Cu + 5 ppb Ag | 5 | 100 |

Exemple III

On a traité une eau contenant 920.000 germes/ml de Staphylocoque aureus ATCC 6538 par diverses compositions. Les résultats de ces traitements sont donnés dans le tableau III suivant :

TABLEAU III

| Quantité et nature des ingrédients ajoutés | temps de contact minutes | % de bactéries restantes |
|---|---|---|
| + 1 ppm Cu<br>+ 5 ppb Ag | 5<br>30 | 100<br>100 |
| 0,5 ppm du composé de l'exemple 10 (comparatif)+ 3 ppm du composé de l'exemple 6 (comparatif) | 5<br>30 | 13,58<br>2,00 |
| idem + 1 ppm Cu + 5 ppb Ag | 5<br>30 | 1,2<br>0,0005 |
| 1,5 ppm polymère exemple 5 (inv.)+ 1,5 ppm polymère exemple 6 (comparatif) | 5 | 6,80 |
| - idem + 1 ppm Cu + 5 ppb Ag | 5 | 0,58 |
| 1,5 ppm polymère exemple 7 (comparatif) + 1,5 ppm polymère exemple 9 (invention) | 5<br>15<br>30 | 16,3<br>1,45<br>0,125 |

Suite du TABLEAU III

| Quantité et nature des ingrédients ajoutés | temps de contact minutes | % de bactéries restantes |
|---|---|---|
| idem + 1 ppm Cu + 5 ppb Ag | 5 | 0,23 |
| | 15 | 0,000 |
| | 30 | 0,000 |
| 1,5 ppm polymère exemple 11 (comparatif) | 5 | 11,95 |
| 1,5 ppm polymère exemple 12 (invention) | 5 | 0,002 |
| | 30 | 0,000 |
| idem + 1 ppm Cu + 5 ppb Ag | 5 | 0,006 |
| | 15 | 0,000 |
| | 30 | 0,000 |

Exemple IV

On a traité une eau contenant 310.000 germes/ml de Streptococus faecalis ATCC 6569 avec des polymères des exemples 1 à 4.

TABLEAU IV

| Quantité et nature des ingrédients ajoutés | temps de contact minutes | % de bactéries restantes |
|---|---|---|
| 3 ppm polymère exemple 2 (invention) + 1 ppm Cu | 5<br>15<br>30 | 16<br>0,023<br>0,0013 |
| 3 ppm polymère exemple 1 (comparatif) | 5<br>15 | 90<br>72 |
| + 1 ppm Cu | 30 | 32 |
| 3 ppm polymère exemple 3 (invention) | 5<br>15 | 2,5<br>0,012 |
| + 1 ppm Cu | 30 | 0,0006 |
| 3 ppm polymère exemple 4 (comparatif) | 5<br>15 | 61<br>32 |
| + 1 ppm Cu | 30 | 5 |

Exemple V

On a isolé une souche bactérienne dans une piscine médicale et on a déterminé que cette souche était un Acinobacter calcoaceticus lwoffi. La concentration initiale en germes était de 135 . 000 germes/ml.

TABLEAU V

| Quantité et nature des ingrédients ajoutés | temps de contact minutes | % de bactéries restantes |
|---|---|---|
| 3 ppm polymère exemple 3 (invention) + 1 ppm Cu | 120 | 0,000 |
| 3 ppm polymère exemple 4 (comparatif) + 1 ppm Cu | 120 | 0,23 |

Exemple VI

On a traité une eau contenant 210 . 000 germes/ml de Acetobacter hansenii déterminé par le Laboratoire J. SIMON - Bierges - Belgique.

Ces germes ont été isolées dans une piscine couverte. Les résultats du traitement de cette eau sont donnés dans le tableau VI suivant :

TABLEAU VI

| Quantité et nature des ingrédients ajoutés | temps de contact | % de bactéries restantes |
|---|---|---|
| 3 ppm polymère exemple 3 (invention) + 1 ppm Cu | 2 heures<br>1 jour<br>6 jours | 17<br>1,04<br>0,000 |
| 3 ppm polymère exemple 4 (comparatif) + 1 ppm Cu | 2 heures<br>1 jour<br>6 jours | 69<br>23<br>4,5 |
| 3 ppm polymère exemple 2 (invention) + 1 ppm Cu | 2 heures<br>1 jour<br>2 jours | 19<br>0,38<br>0,000 |
| 3 ppm polymère exemple 1 (comparatif) + 1 ppm Cu | 2 heures<br>1 jour<br>2 jours | 137<br>231<br>461 |
| 3 ppm polymère exemple 13 (comparatif) + 1 ppm Cu | 2 heures<br>1 jour<br>2 jours | 105<br>98<br>117 |

De plus, on a remarqué que le traitement desdits germes par les polymères des exemples 1 et 13 (comparatifs) provoquait le dégagement d'odeurs nauséabondes.

Exemple VII

On a traité une eau contenant des germes de Pseudomonas Stujeri provenant d'une piscine avec 3 ppm d'un composé de formule :

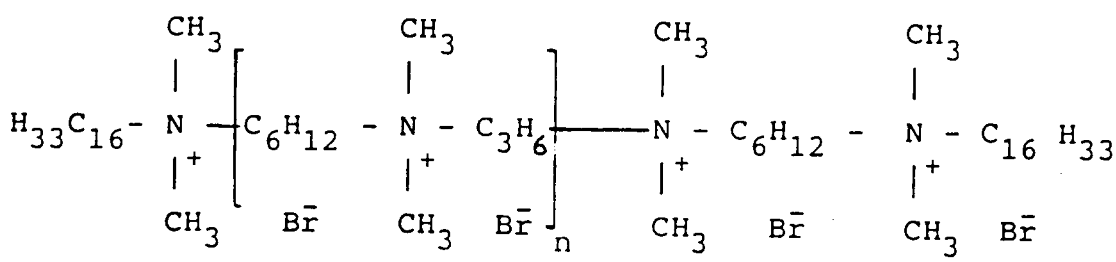

et 1 ppm d'ion Cu.

Les résultats de ces essais sont donnés dans le tableau suivant :

| n | poids moléculaire | % de bactéries restantes après 120 minutes |
|---|---|---|
| 4 (invention) | 2280 | 0,00004 |
| 5 (invention) | 2654 | 0,00004 |
| 12 (comparatif) | 5300 | 0,001 |
| 19 (comparatif) | 7900 | 1,2 |

Exemple VIII

De manière similaire à l'exemple VIII, une eau de piscine contenant des germes d'Acetobacter Hansenii a été traitée par 3 ppm d'un composé de formule

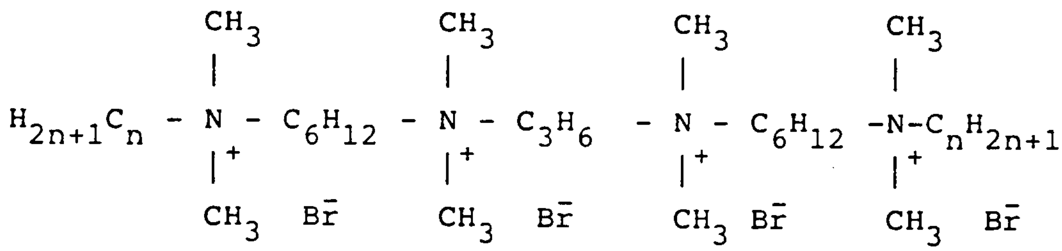

et 1 ppm de Cu.

Les résultats de ces essais sont donnés dans le tableau suivant :

| composé | n | % des germes restants après 2 heures | |
|---|---|---|---|
| | | hansenii | lwoffi |
| H (comparatif) | 8 | 30 | 50 |
| J (invention) | 12 | 0,004 | 0,003 |
| K (invention) | 16 | 0,022 | 0,0009 |

<u>Exemple IX</u>

Après stérélisation de filtres en papier Whatman® (qualité 4), on a traité ceux-ci au moyen de diverses solutions. Le tableau suivant donne les traitements subis par les différents papiers-filtre.

| Filtre | Traitement |
|---|---|
| A | Aucun |
| B | le filtre a été plongé pendant 10 minutes dans une solution à 1 % du composé K de l'exemple IX et a ensuite été rincé 3 fois avec 100 ml d'eau |
| C | le filtre a été plongé pendant 10 minutes dans une solution à 1 % du composé K de l'exemple IX et à 0,01 % $Ag^+$ et rincé trois fois avec 100 ml d'eau distillée. |

On a préparé une suspension de Staphylococcus Aureus (ATCC 6538) contenant environ 2 $10^4$ germes/ml.

On a placé 1 ml de cette suspension sur un milieu tryptone-glucose-extrait agar. Après avoir déposé sur ledit milieu le papier on a placé ledit milieu dans une chambre d'incubation (37° C).

On a ainsi remarqué que les bactéries croissent dans le milieu ainsi que sur le filtre A, tandis que la croissance des bactéries est empechée sur les filtres B et C.

Ainsi les produits et compositions suivant l'invention adhérent au papier et ne diffusent pas dans le milieu. Un papier traité par une composition suivant l'invention peut dès lors être considéré comme une barrière efficace au passage de bactéries.

Plus de 72 heures après le traitement, le papier filtre traité possédait toujours des propriétés antibactériennes.

<u>Exemple X</u>

On a traité une eau de piscine contenant 540 000 germes/ml d'Acinetobacter calcoaceticus lwoffi avec 2 ppm d'un composé choisi parmi les composés des exemples 30 à 33 et 1 ppm de Cu et une eau de piscine contenant 440 000 germes/ml de Pseudomonas stuzeri avec 2 ppm d'un composé choisi parmi les composés des exemples 30 à 33 en combinaison avec 10 ppb d'argent.

Ces essais ont montré que l'Acinetobacter calcoaceticus et le Pseudomonas stutzeri étaient détruits très rapidement. Ainsi, au plus tard 4 heures après le début du traitement, moins de 0,00001 % du nombre de germes présents au début du traitement restait dans le milieu.

Des essais sur des souches de champignons que l'on retouve aux abords des piscines telles que Trichophyton rubrum ou Chrysosporium Keratinophilium ont montré l'action fongicide en 24 heures du composé de l'exemple 30 dissous dans l'eau à une concentration moindre que 50 ppm en présence de 30 ppm de Cu et 100 ppb de Ag.

L'invention a donc aussi pour objet une composition fongicide à usage externe par exemple pour le traitement de champignons se développant sur le corps humain ou sur le corps d'un animal, cette composition contenant au moins un composé suivant l'invention et, de préférence, un agent favorisant la pénétration dudit composé. Un tel agent est par exemple de l'éthanol ou un autre alcool.

Dans le cas où la composition ou le procédé suivant l'invention sont appliqués à la désinfection d'eau, on utilise, de préférence au moins un polymère d'ammonium quaternaire de formule 1 tel que défini plus haut, à raison de 0,5 à 1000 parties par million (ppm) du milieu aqueux à désinfecter. Quant aux ions des métaux, tels que le cuivre et l'argent, qui peuvent être produits au sein de ce milieu aqueux par électrolyse ou par addition à celui-ci de sels hydrosolubles de ces métaux, tels que sulfate, chlorure, nitrate, etc, ils sont utilisés, de préférence, à des concentrations de 0,5 à 5 ppm pour les ions de cuivre et de 1 à 50 ppb (parties par milliard), plus particulièrement de 1 à 10 ppb pour les ions d'argent.

Lorsqu'on doit désinfecter des surfaces, la composition suivant l'invention est projetée ou pulvérisée sur lesdites surfaces.

Il va de soi que pour désinfecter des surfaces, il est également possible de tremper les surfaces à désinfecter dans un bain.

La composition suivant l'invention est également utile pour la conservation d'aliments ou de boissons puisqu'elle permet une destruction rapide des germes au moyen de très faible quantité et qu'elle reste efficace pendant une longue période de temps.

On a préparé une composition similaire à celle de l'exemple 5, si ce n'est que l'on a utilisé une eau minérale vendue en bouteilles de 1,5 litre pour la préparation d'une solution contenant ladite composition et pour la dilution de ladite solution.

Cette solution diluée additionnée de cuivre et d'argent a ensuite été ajoutée dans des bouteilles d'eau vendues dans le commerce de sorte que l'eau contenait moins de 1 ppm de polymère, de préférence environ 0,5 ppm de polymère, environ 0,4 ppm de cuivre et 5 ppb d'argent.

Les résultats de l'utilisation de cette composition pour la conservation de boissons sont donnés dans le tableau VII suivant :

TABLEAU VII

| Quantité et nature des ingrédients ajoutés | x heures | nombre de germes restants (type pseudomonas) après x heures |
|---|---|---|
| eau de commerce | | |
| + 0,5 ppm polymère exemple 5 (invention)<br>+ 0,4 ppm cuivre<br>+ 5 ppm argent | 0<br>2<br>2000 | 3000<br>0<br>0 |
| eau de commerce | 0<br>2<br>2000 | 3000<br>5000<br>6000 |

La composition suivant l'invention peut, vu la faible quantité nécessaire pour obtenir la destruction rapide des germes, être utilisée dans la conservation de boissons, d'aliments.

La composition suivant l'invention peut ainsi être ajoutée pendant l'étape de cuisson de confitures, peut être injectée dans des aliments. On peut également imprégner ou imbiber des aliments d'une composition suivant l'invention en trempant lesdits aliments dans un bain contenant ladite composition ou en pulvérisant sur lesdits aliments ladite composition.

De façon avantageuse, la composition suivant l'invention peut être utilisée pour le dégraissage et la stérilisation de la laine.

Les composés suivant l'invention peuvent être utilisés dans des savons, dentifrices, shampooings, pansements médicaux, pour la conservation d'aiguilles de seringues, de verres de contact, pour la stérilisation d'enzymes extraits de bactéries sans risque de dénaturation, pour la protection du bois, pour la conservation d'hydrocarbures, pétrole, papiers, cotons, pour la destruction de bactéries sulfato-réductrices, pour des traitements agricoles, antifongiques.

## Revendications

**1.** Composé d'ammonium quaternaire répondant à la formule suivante :

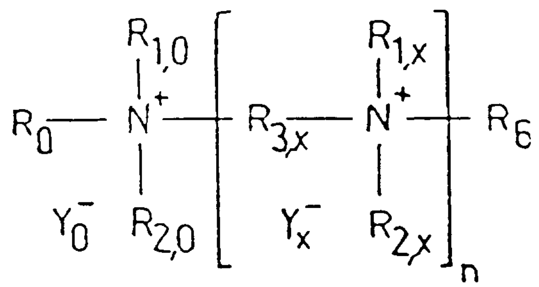

(1)

dans laquelle :

n est un nombre entier supérieur ou égal à 1;

x est un nombre compris entre 1 et n :

$R_o$ et $R_6$ qui peuvent être identiques ou différents désignent un radical hydrocarboné éventuellement insaturé, ce radical contenant de 1 a 22 atomes de carbone;

$R_{1,y}$, $R_{2,y}$ pour y compris entre 0 et n désignent un groupe hydrocarboné éventuellement ramifié, insaturé et/ou substitué par un ou plusieurs halogènes ou par un groupement hydrocarboné, $R_{1,y}$ et $R_{2,y}$ pouvant contenir jusqu'à 22 atomes de carbone; au moins un radical $R_o$, $R_6$, $R_{1,y}$ ou

$R_{2,y}$ désigne un radical contenant de 10 à 22 atomes de carbone;

$R_{3,x}$, pour x compris entre 1 et n, désigne un groupe hydrocarboné éventuellement insaturé et/ou ramifié pouvant contenir jusqu'à 10 atomes de carbone, ou un groupe de formule :

$- (CH_2)_s - S - (CH_2)_t-$
$- (CH_2)_s - S - S - (CH_2)_t-$
$- (CH_2)_s - S\ 0 - (CH_2)_t-$

où s et t sont des nombres entiers,

ces groupes étant éventuellement substitués par des

groupements hydrocarbonés,

$R_{3,x}$ pouvant être identique ou différent de $R_{3,x+1}$ , et

$Y_o^-$ et $Y_x^-$ désignent un anion, de préférence, un atome d'halogène, le poids moléculaire de ce composé étant compris entre 1000 et 2654

ou répondant à l'une quelconque des formules suivantes :

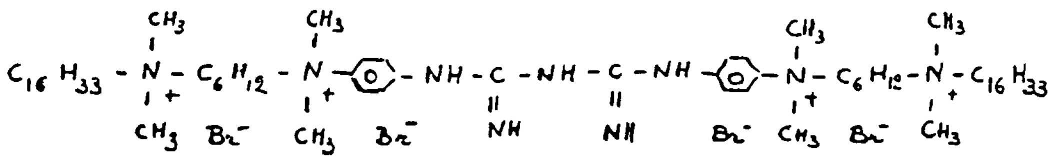

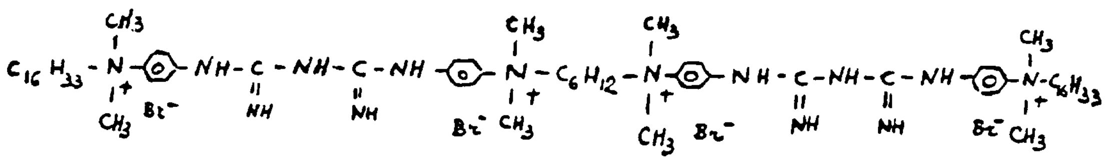

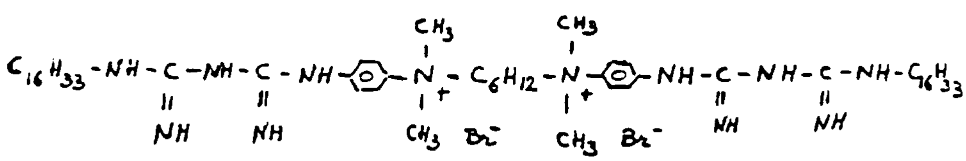

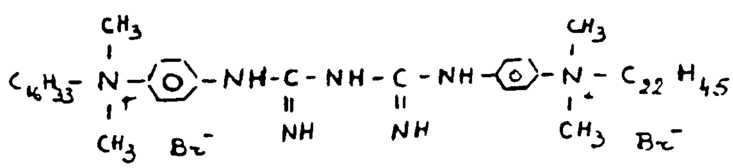

2. Composé suivant la revendication 1, caractérise en ce que $R_o$ et $R_6$ sont identiques et contiennent de 10 à 22 atomes de carbone.

3. Composé suivant la revendication 1, caractérisé en ce qu'au moins un radical $R_o$, $R_6$, $R_{1,y}$ ou $R_{2,y}$ contient de 12 à 16 atomes de carbone.

4. Composé suivant l'une quelconque des revendications 1 à 3, caractérisé en ce que $R_{3,x}$ est un groupe de formule $-CH_2-CH_2-CH_2-$ lorsque x est un nombre pair, tandis que $R_{3,x}$ est un groupe de formule $-(CH_2)_6-$ lorsque x est un nombre impair, ou inversément.

5. Composé suivant la revendication 1, caractérisé en ce que s et t sont des nombres entiers compris entre 1 et 3.

6. Composé suivant la revendication 1, caractérisé en ce que pour au moins un x compris entre 1 et n, $R_{3,x}$ désigne un groupe

$-(CH_2)_w-$ avec w compris entre 1 et 10 ;

ou

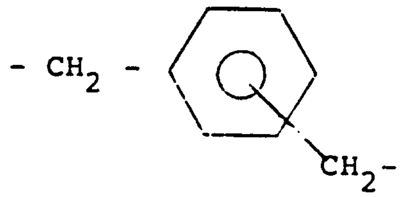

,

un des substituants $-CH_2-$ pouvant être en position ortho, méta ou para ; ou

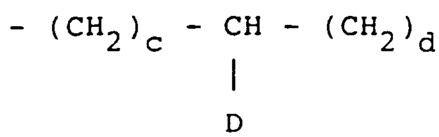

dans laquelle D désigne un atome d'hydrogène ou un radical $C_{1-4}$ alkyl,

c et d sont des nombres entiers inférieurs à 5 dont l'un peut avoir une valeur égale à 0, tandis que la somme c + d est au moins égale à 1 et au maximum égale à 8,

le radical pouvant contenir au maximum deux doubles liaisons ; ou

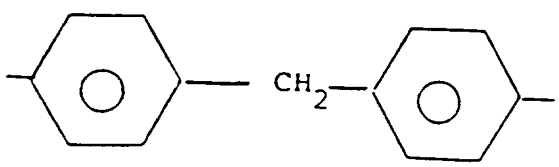

**7.** Composition destinée à la désinfection de liquides et/ou surfaces ou à la conservation d'aliments ou boissons, caractérisée en ce qu'elle contient au moins un composé suivant l'une quelconque des revendications précédentes.

**8.** Composition suivant la revendication 7, caractérisée en ce qu'elle contient au moins un ion d'un métal choisi parmi le fer, le cuivre, l'argent ou un mélange de tels ions.

**9.** Composition suivant l'une quelconque des revendications 7 et 8, caractérisée en ce qu'elle contient également un deuxième composé d'ammonium quaternaire de formule générale

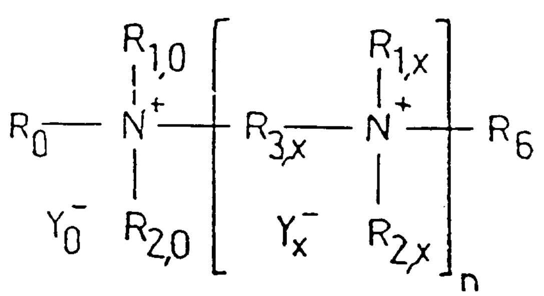

(2)

dans laquelle :

n est un nombre entier supérieur ou égal à 1 ;

x est un nombre compris entre 1 et n :

$R_o$ et $R_6$ qui peuvent être identiques ou différents désignent un radical hydrocarboné éventuellement insaturé, ce radical contenant de 1 à 22 atomes de carbone , et

$R_{1,y}$, $R_{2,y}$ pour y compris entre 0 et n désignent un groupe hydrocarboné éventuellement ramifié, insaturé et/ou substitué par un ou plusieurs halogènes ou par un groupement hydrocarboné, $R_{1,y}$ et $R_{2,y}$ pouvant contenir jusqu'à 22 atomes de carbone,

$Y_o^-$ et $Y_x^-$ désignent un anion, de préférence un atome d'halogène, et

$R_{3,x}$ désigne un groupe hydrocarboné éventuellement insaturé et/ou ramifié pouvant contenir jusqu'à 10 atomes de carbone, ou

un groupe de formule :

$- (CH_2)_s - 0 - (CH_2)_t-$
$- (CH_2)_s - S - (CH_2)_t-$
$- (CH_2)_s - S - S - (CH_2)_t-$
$- (CH_2)_s - S\ 0 - (CH_2)_t-$

où s et t sont des nombres entiers,

ces groupes étant éventuellement substitués,

le poids moléculaire de ce composé étant inférieur à 5000, et, de préférence, compris entre 1000 et 4500.

**10.** Composition suivant la revendication 9, caractérisée en ce qu'au moins un radical désigné par $R_o$, $R_6$, $R_{1,y}$ ou $R_{2,y}$ du deuxième composé d'ammonium quaternaire désigne un radical contenant de 10 à 22 atomes de carbone.

**11.** Composition suivant l'une quelconque des revendications 9 et 10, caractérisée en ce que $R_o$ et $R_6$ du deuxième composé d'ammonium désignent un radical contenant de 12 à 16 atomes de carbone.

**12.** Composition suivant la revendication 11, caractérisée en ce que $R_o$ et $R_6$ contiennent un même nombre d'atomes de carbone.

**13.** Composition suivant la revendication 12, caractérisée en ce que $R_o$ et $R_6$ contiennent 16 atomes de carbone.

**14.** Composition suivant l'une quelconque des revendications 9 à 13, caractérisée en ce que $R_{3,x}$ du deuxième composé d'ammonium est une groupe de formule $- CH_2-CH_2-CH_2-$ lorsque x est un nombre pair, tandis que $R_{3,x}$ est un groupe de formule $-(CH_2)_6-$ lorsque x est un nombre impair, ou inversément.

**15.** Composition suivant la revendication 9 ou 10, caractérisée en ce que s et t sont des nombres entiers compris entre 1 et 3.

**16.** Composition suivant la revendication 9 ou 10, caractérisée en ce que pour au moins un x compris entre 1 et n, $R_{3,x}$ désigne un groupe
$- (CH_2)_w -$ avec w compris entre 1 et 10 ;
ou

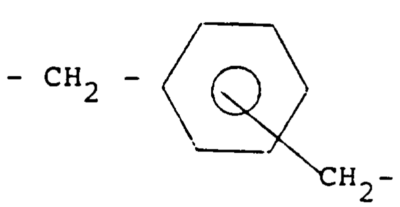

,

un des
substituants $-CH_2-$ pouvant être en position ortho, méta ou para ; ou

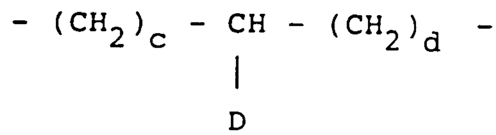

dans laquelle D désigne un atome d'hydrogène ou un radical $C_{1-4}$ alkyl,
c et d sont des nombres entiers inférieurs à 5 dont l'un peut avoir une valeur égale à 0, tandis que la somme c + d est au moins égale à 1 et au maximum égale à 8,
le radical pouvant contenir au maximum deux doubles liaisons ; ou

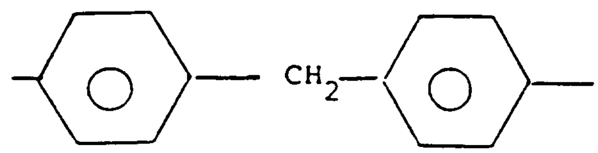

**17.** Composition suivant la revendication 16, caractérisée en ce que $R_{1,v}$ et $R_{2,v}$ du deuxième composé d'ammonium pour un v compris entre 0 et n désignent un radical alkyl inférieur, de préférence $-CH_3$,

**18.** Composition suivant l'une quelconque des revendications 9 à 16, caractérisée en ce que le rapport en poids du premier composé de formule 1 ayant un poids moléculaire compris entre 1000 et 2654 et du composé de formule 2 ayant un poids moléculaire inférieur à 5000 est compris entre 0,1 et 10, de préférence entre 0,5 et 6.

**19.** Composition suivant la revendication 18, caractérisée en ce que le composé de formule 2 a un poids moléculaire compris entre 1000 et 4500, le rapport en poids de ces composés étant d'environ 1.

**20.** Procédé de désinfection de liquides, caractérisé en ce qu'on ajoute à ces liquides au moins une composition suivant l'une quelconque des revendications 7 à 19.

**21.** Procédé de désinfection de surfaces infectées, caractérisé en ce qu'on met ces surfaces en contact avec au moins une composition désinfectante suivant l'une quelconque des revendications 7 à 19.

**22.** Procédé de conservation de boissons dans lequel on ajoute auxdites boissons une composition suivant l'une quelconque des revendications 7 à 19.

**23.** Procédé de conservation d'aliments, dans lequel on injecte dans lesdits aliments une composition suivant l'une quelconque des revendications 7 à 19.

**24.** Procédé de conservation d'aliments, dans lequel on trempe lesdits aliments dans une composition suivant l'une quelconque des revendications 7 à 19.

**25.** Support traité par une composition suivant l'une quelconque des revendications 7 à 19.

**26.** Composition fongicide et/ou bactéricide, caractérisée en ce qu'elle contient au moins un composé suivant l'une quelconque des revendications 1 à 6.

## Claims

**1.** Compound of quaternary ammonium according to the formula :

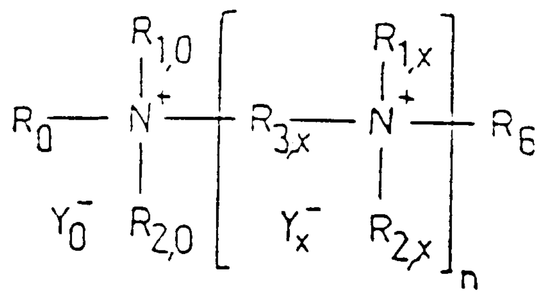

(1)

wherein :
n is an integer higher than or equal to 1 ;
x is a number comprised between 1 and n ;
$R_0$ and $R_6$, which may be identical or different, refer to a hydrocarbon radical possibly unsaturated, said radical containing from 1 to 22 carbon atoms ;
$R_{1,y}$ and $R_{2,y}$, for y comprised between 0 and n, refer to a hydrocarbon group possibly ramified, unsaturated and/or substituted by one or more halogen atoms or by a hydrocarbon group, whereby $R_{1,y}$ and $R_{2,y}$ may contain up to 22 carbon atoms; at least one radical $R_0$, $R_6$, $R_{1,y}$ or $R_{2,y}$ refer to a radical containing 10 to 22 carbon atoms ;
$R_{3,x}$, for x comprised between 1 and n, refers to a possibly unsaturated and/or ramified hydrocarbon group which may contain up to 10 carbon atoms, or a group of formula :

$-(CH_2)_s-S-(CH_2)_t-$
$-(CH_2)_s-S-S-(CH_2)_t-$
$-(CH_2)_s-S\ O-(CH_2)_t-$

wherein s and t are integers,
these groups being possibly substituted by hydrocarbon groups,
$R_{3,x}$ may be identical to or different from $R_{3,x+1}$ ; and
$Y_0^-$ and $Y_x^-$ refer to an anion, preferably an halogen atom, the molecular weight of this compound being comprised between 1000 and 2654;
or having anyone of the following formulae :

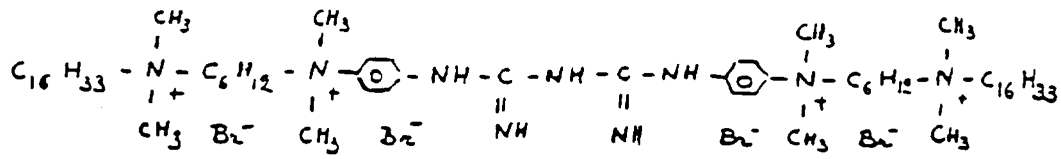

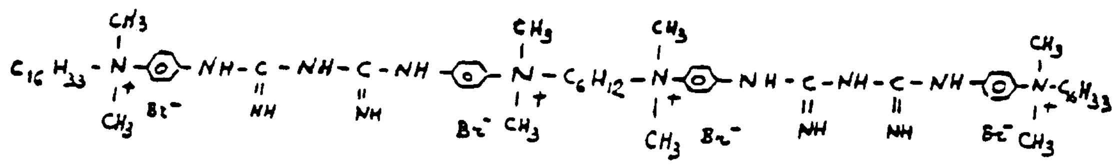

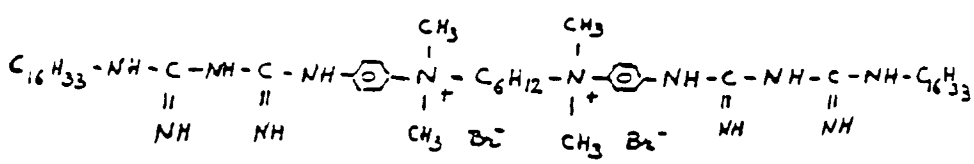

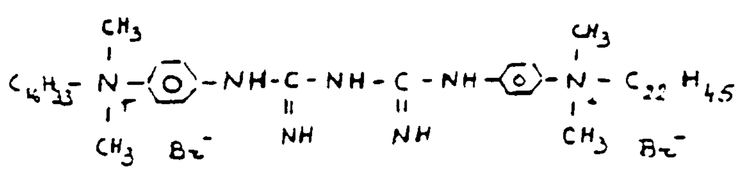

2. Compound according to claim 1, characterized in that $R_0$ and $R_6$ are identical and contain from 10 to 22 carbon atoms.

3. Compound according to claim 1 or 2, characterized in that at least one radical $R_0$, $R_6$, $R_{1,y}$ or $R_{2,y}$ contains from 12 to 16 carbon atoms.

4. Compound according to anyone of the claims 1 to 3, characterized in that $R_{3,x}$ is a group of the formula $-CH_2-CH_2-CH_2-$ when x is an even number, whereas $R_{3,x}$ is a group of the formula $-(CH_2)_6-$ when x is an odd number, or inversely.

5. Compound according to claim 1, characterized in that s and t are integers comprised between 1 and 3.

6. Compound according to claim 1, characterized in that for at least one x comprised between 1 and n, $R_{3,x}$ refers to a group
$-(CH_2)_w-$ with w comprised between 1 and 10 ;
or

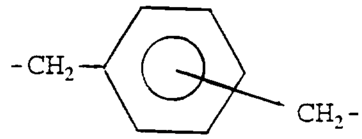

in which one of the $-CH_2-$ substituents may be in the ortho, meta or para position ; or

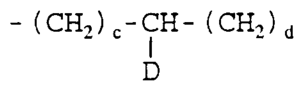

wherein D refers to an hydrogen atom or a $C_{1-4}$ alkyl radical,
c and d are integers lower than 5, one of which may be equal to 0 while the sum c + d is at least equal to 1 and at most equal to 8,
whereby the radical may contain at most two double bonds ; or

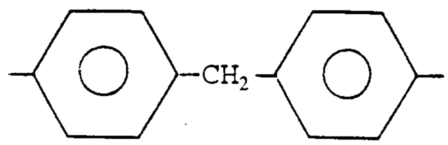

**7.** Composition intended for disinfecting liquids and/or surfaces or for preserving food or drinks, characterized in that it contains at least one compound according to anyone of the preceding claims.

**8.** Composition according to claim 7, characterized in that it contains at least one ion of a metal selected among iron, copper, silver or a mixture of such ions.

**9.** Composition according to anyone of the claims 7 and 8, characterized in that it also contains a second quaternary ammonium compound of the general formula

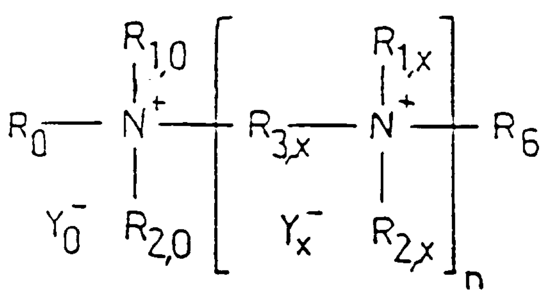

(2)

wherein :
n is an integer greater than or equal to 1 ;
x is a number comprised between 1 and n ;
$R_0$ and $R_6$, which may be identical or different, refer to a hydrocarbon radical possibly unsaturated, said radical containing from 1 to 22 carbon atoms, and
$R_{1,y}$ and $R_{2,y}$, for y comprised between 0 and n, refer to a hydrocarbon group possibly ramified, unsaturated and/or substituted by one or more halogen atoms or by a hydrocarbon group, whereby $R_{1,y}$ and $R_{2,y}$ may contain up to 22 carbon atoms ;
$Y_0^-$ and $Y_x^-$ refer to an anion, preferably an halogen atom, and

$R_{3,x}$ refers to a hydrocarbon group possibly unsaturated and/or ramified which may contain up to 10 carbon atoms, or a group of formula :

$-(CH_2)_s-O-(CH_2)_t-$
$-(CH_2)_s-S-(CH_2)_t-$
$-(CH_2)_s-S-S-(CH_2)_t-$
$-(CH_2)_s-S\ O-(CH_2)_t-$

wherein s and t are integers,
these groups being possibly substituted,
the molecular weight of this compound being lower than 5000 and, preferably, comprised between 1000 and 4500.

**10.** Composition according to claim 9, characterized in that at least one radical referred to by $R_0$, $R_6$, $R_{1,y}$ or $R_{2,y}$ of the second quaternary ammonium compound, designates a radical containing from 10 to 22 carbon atoms.

**11.** Composition according to anyone of the claims 9 and 10, characterized in that $R_0$ and $R_6$ from the second ammonium compound refer to a radical that contains from 12 to 16 carbon atoms.

**12.** Composition according to claim 11, characterized in that $R_0$ and $R_6$ both comprise the same number of carbon atoms.

**13.** Composition according to claim 12, characterized in that $R_0$ and $R_6$ both comprise 16 carbon atoms.

**14.** Composition according to anyone of the claims 9 to 13, characterized in that the group $R_{3,x}$ of the second ammonium compound has the formula $-CH_2-CH_2-CH_2-$ when x is an even number, whereas the group $R_{3,x}$ has the formula $-(CH_2)_6-$ when x is an odd number, or inversely.

**15.** Composition according to claim 9 or 10, characterized in that s and t are integers comprised between 1 and 3.

**16.** Composition according to claim 9 or 10, characterized in that for at least one x comprised between 1 and n, $R_{3,x}$ refer to a group
$-(CH_2)_w-$, with w being comprised between 1 and 10 ;
or

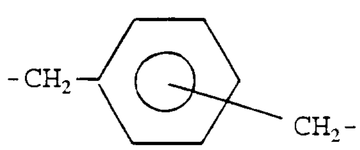

whereby one of the $-CH_2-$ substituents may be in the ortho, meta or para position ; or

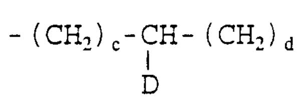

wherein D is an hydrogen atom or a $C_{1-4}$ alkyl radical,
c and d are integers lower than 5, one of which may be equal to 0, while the sum c + d is at least equal to 1 and at most equal to 8,
whereby the radical may comprise a maximum of two double bonds ; or

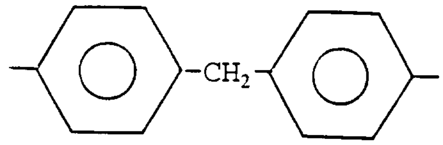

**17.** Composition according to claim 16, characterized in that for one v comprised between 0 and n, $R_{1,v}$ and $R_{2,v}$ of the second ammonium compound refer to a lower alkyl radical, preferably $-CH_3$.

**18.** Composition according to anyone of the claims 9 to 16, characterized in that the weight ratio of the first compound having the formula 1 and a molecular weight comprised between 1000 and 2654 to the compound having the formula 2 and a molecular weight lower than 5000, is comprised between 0.1 and 10, preferably between 0.5 and 6.

**19.** Composition according to claim 18, characterized in that the compound having the formula 2 has a molecular weight comprised between 1000 and 4500, the weight ratio of these compounds being about 1.

**20.** Process for disinfecting liquids, characterized in that at least one composition according to anyone of the claims 7 to 19 is added to said liquids.

**21.** Process for disinfecting contaminated surfaces, characterized in that said surfaces are contacted with at least one disinfecting composition according to anyone of the claims 7 to 19.

**22.** Process for preserving drinks, characterized in that a composition according to anyone of the claims 7 to 19 is added to said drinks.

**23.** Process for preserving food, characterized in that a composition according to anyone of the claims 7 to 19 is injected into said food.

**24.** Process for preserving food, characterized in that said food is soaked in a composition according to anyone of the claims 7 to 19.

**25.** Support treated with a composition according to anyone of the claims 7 to 19.

**26.** Fungicidal and/or bactericidal composition, characterized in that it comprises at least one compound according to anyone of the claims 1 to 6.

**Patentansprüche**

**1.** Quartäre Ammoniumverbindung, die der folgenden Formel entspricht:

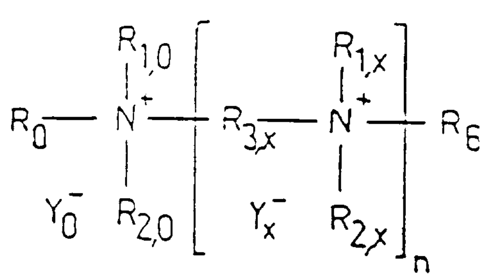

(1)

in welcher:
n eine ganze Zahl größer oder gleich 1 ist;

x eine Zahl zwischen 1 und n ist;

$R_o$ und $R_6$, die gleich oder verschieden sein können, einen gegebenenfalls ungesättigten Kohlenwasserstoffrest bezeichnen, wobei der Rest 1 bis 22 Kohlenstoffatome enthält;

$R_{1,y}$ und $R_{2,y}$, in welchen y zwischen 0 und n beträgt, eine gegebenenfalls verzweigte, ungesättigte und/oder mit einem oder mehreren Halogenatom(en) oder mit einer Kohlenwasserstoffgruppierung substituierte Kohlenwasserstoffgruppe bezeichnen und $R_{1,y}$ und $R_{2,y}$ bis zu 22 Kohlenstoffatome enthalten können;

mindestens ein Rest $R_o$, $R_6$, $R_{1,y}$ oder $R_{2,y}$ einen Rest bezeichnet, welcher 10 bis 22 Kohlenstoffatome enthält;

$R_{2,y}$ einen Rest, welcher 10 bis 22 Kohlenstoffatome enthält, bezeichnet;

$R_{3,x}$, in welchem x zwischen 1 und n beträgt, eine gegebenenfalls ungesättigte und/oder verzweigte Kohlenwasserstoffgruppe bezeichnet, welche bis zu 10 Kohlenstoffatome enthalten kann, oder eine Gruppe der Formel:

$- (CH_2)_s - S - (CH_2)_t-$
$- (CH_2)_s - S - S - (CH_2)_t-$
$- (CH_2)_s - S\ 0 - (CH_2)_t-$

in welcher s und t ganze Zahlen sind,

wobei diese Gruppen gegebenenfalls mit Kohlenwasserstoffgruppierungen substituiert sind,

$R_{3,x}$ gleich oder verschieden von $R_{3,x+1}$ sein kann, und

$Y_o^-$ und $Y_x^-$ ein Anion, vorzugsweise ein Halogenatom, bezeichnen, wobei das Molekulargewicht dieser Verbindung zwischen 1000 und 2654 liegt,

oder welche einer der folgenden Formeln entspricht:

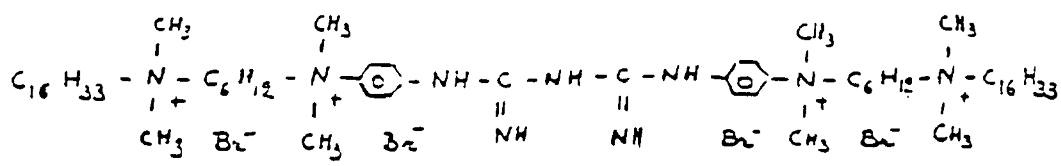

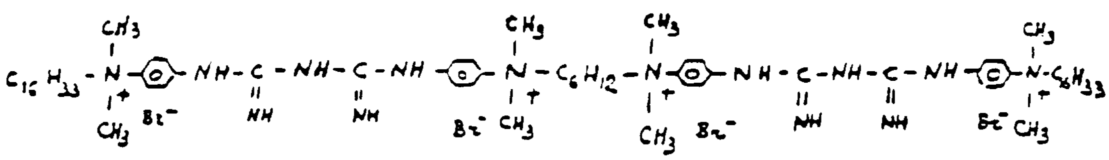

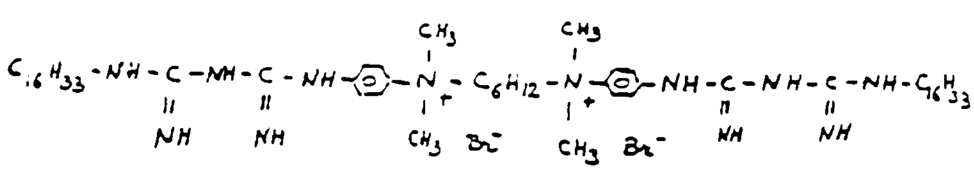

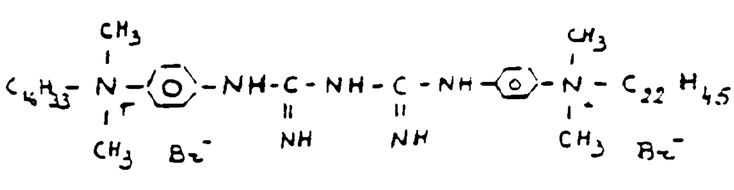

2. Verbindung nach Anspruch 1,
dadurch gekennzeichnet,
daß $R_o$ und $R_6$ gleich sind und 10 bis 22 Kohlenstoffatome enthalten.

3. Verbindung nach Anspruch 1,
dadurch gekennzeichnet,
daß mindestens ein Rest $R_o$, $R_6$, $R_{1,y}$ oder $R_{2,y}$ 12 bis 16 Kohlenstoffatome enthält.

4. Verbindung nach irgendeinem der Ansprüche 1 bis 3,
dadurch gekennzeichnet,
daß $R_{3,x}$ eine Gruppe der Formel $-CH_2-CH_2-CH_2-$ ist, wenn x eine gerade Zahl ist, wohingegen $R_{3,x}$ eine Gruppe der Formel $-(CH_2)_6-$ ist, wenn x eine ungerade Zahl ist, oder vice versa.

5. Verbindung nach Anspruch 1,
dadurch gekennzeichnet,
daß s und t ganze Zahlen zwischen 1 und 3 sind.

6. Verbindung nach Anspruch 1,
dadurch gekennzeichnet,
daß, wenn mindestens ein x zwischen 1 und n beträgt, $R_{3,x}$ eine Gruppe bezeichnet
$-(CH_2)_w-$, wobei w zwischen 1 und 10 beträgt,
oder

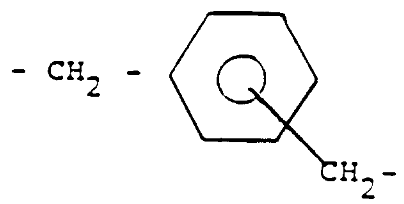

wobei einer der $(-CH_2-)$-Substituenten in ortho-, meta- oder para-Position sein kann,
oder

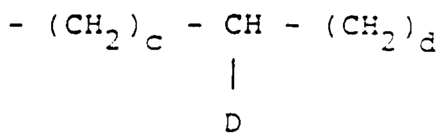

in welcher:
D ein Wasserstoffatom oder einen $C_{1-4}$-Alkylrest bezeichnet,
c und d ganze Zahlen kleiner als 5 sind, von denen einer einen Wert gleich 0 haben kann, wohingegen die Summe c + d mindestens gleich 1 und höchstens gleich 8 ist,
der Rest höchstens zwei Doppelbindungen enthalten kann, oder

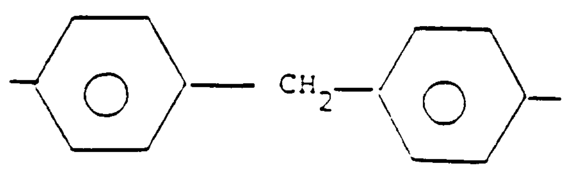

7. Zusammensetzung zur Desinfektion von Flüssigkeiten und/oder Oberflächen oder zur Konservierung von Lebensmitteln oder Getränken,
dadurch gekennzeichnet,
daß sie mindestens eine Verbindung nach irgendeinem der vorangegangenen Ansprüche enthält.

8. Zusammensetzung nach Anspruch 7,
dadurch gekennzeichnet,
daß sie mindestens ein Ion eines Metalls, ausgewählt aus Eisen, Kupfer und Silber, oder eine Mischung dieser Ionen enthält.

**9.** Zusammensetzung nach irgendeinem der Ansprüche 7 und 8,
dadurch gekennzeichnet,
daß sie gleichfalls eine zweite quartäre Ammoniumverbindung der allgemeinen Formel enthält:

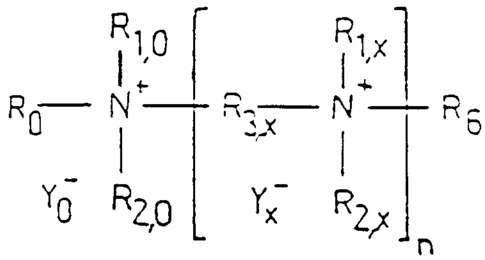

(2)

in welcher:
n eine ganze Zahl größer oder gleich 1 ist;
x eine Zahl zwischen 1 und n ist;
$R_o$ und $R_6$, die gleich oder verschieden sein können, einen gegebenenfalls ungesättigten Kohlenwasserstoffrest bezeichnen, wobei der Rest 1 bis 22 Kohlenstoffatome enthält, und
$R_{1,y}$ und $R_{2,y}$, in welchen y zwischen 0 und n liegt, eine gegebenenfalls verzweigte, ungesättigte und/oder mit einem oder mehreren Halogenatom(en) oder mit einer Kohlenwasserstoffgruppierung substituierte Kohlenwasserstoffgruppe bezeichnen und $R_{1,y}$ und $R_{2,y}$ bis zu 22 Kohlenstoffatome enthalten können,
$Y_o^-$ und $Y_x^-$ ein Anion, vorzugsweise ein Halogenatom, bezeichnen, und
$R_{3,x}$ eine gegebenenfalls ungesättigte und/oder verzweigte Kohlenwasserstoffgruppe bezeichnet, welche bis zu 10 Kohlenstoffatome enthalten kann, oder eine Gruppe der Formel:

$-(CH_2)_s - 0 - (CH_2)_t-$
$-(CH_2)_s - S - (CH_2)_t-$
$-(CH_2)_s - S - S - (CH_2)_t-$
$-(CH_2)_s - S\ 0 - (CH_2)_t-$

in welcher s und t ganze Zahlen sind,
wobei diese Gruppen gegebenenfalls substituiert sind,
wobei das Molekulargewicht dieser Verbindung niedriger als 5000 ist und vorzugsweise zwischen 1000 und 4500 liegt,

**10.** Zusammensetzung nach Anspruch 9,
dadurch gekennzeichnet,
daß mindestens ein als $R_o$, $R_6$, $R_{1,y}$ oder $R_{2,y}$ bezeichneter Rest der zweiten quartären Ammoniumverbindung einen Rest, welcher 10 bis 22 Kohlenstoffatome enthält, bezeichnet.

**11.** Zusammensetzung nach irgendeinem der Ansprüche 9 und 10,
dadurch gekennzeichnet,
daß $R_o$ und $R_6$ der zweiten quartären Ammoniumverbindung einen Rest, welcher 12 bis 16 Kohlenstoffatome enthält, bezeichnen.

**12.** Zusammensetzung nach Anspruch 11,
dadurch gekennzeichnet,
daß $R_o$ und $R_6$ eine gleiche Anzahl Kohlenstoffatome enthalten.

**13.** Zusammensetzung nach Anspruch 12,
dadurch gekennzeichnet,
daß $R_o$ und $R_6$ 16 Kohlenstoffatome enthalten.

**14.** Zusammensetzung nach irgendeinem der Ansprüche 9 bis 13, dadurch gekennzeichnet, daß $R_{3,x}$ der zweiten Ammoniumverbindung eine Gruppe der Formel $-CH_2-CH_2-CH_2-$ ist, wenn x eine gerade Zahl ist, wohingegen $R_{3,x}$ eine Gruppe der Formel $-(CH_2)_6-$ ist, wenn x eine ungerade Zahl ist, oder vice versa.

**15.** Zusammensetzung nach Anspruch 9 oder 10, dadurch gekennzeichnet, daß s und t ganze Zahlen zwischen 1 und 3 sind.

**16.** Verbindung nach Anspruch 9 oder 10, dadurch gekennzeichnet, daß, wenn mindestens ein x zwischen 1 und n beträgt, $R_{3,x}$ eine Gruppe bezeichnet $-(CH_2)_w-$, wobei w zwischen 1 und 10 beträgt, oder

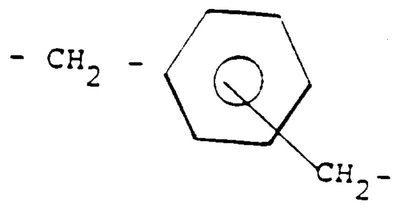

wobei einer der ($-CH_2-$)-Substituenten in ortho-, meta- oder para-Position sein kann, oder

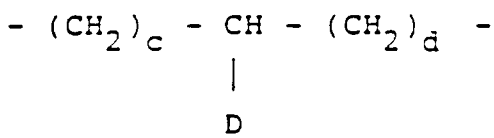

in welcher:
D ein Wasserstoffatom oder einen $C_{1-4}$-Alkylrest bezeichnet,
c und d ganze Zahlen kleiner als 5 sind, von denen einer einen Wert gleich 0 haben kann, wohingegen die Summe c + d mindestens gleich 1 und höchstens gleich 8 ist,
der Rest höchstens zwei Doppelbindungen enthalten kann, oder

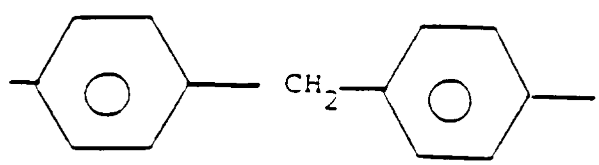

**17.** Zusammensetzung nach Anspruch 16, dadurch gekennzeichnet, daß $R_{1,v}$ und $R_{2,v}$ der zweiten Ammoniumverbindung, wobei ein v zwischen 0 und n beträgt, einen niedrigeren Alkylrest, vorzugsweise $-CH_3$, bezeichnen.

**18.** Zusammensetzung nach irgendeinem der Ansprüche 9 bis 16, dadurch gekennzeichnet, daß das Gewichtsverhältnis der ersten Verbindung der Formel 1, deren Molekulargewicht zwischen 1000 und 2654 liegt, und der Verbindung der Formel 2, deren Molekulargewicht weniger als 5000 beträgt, zwischen 0,1 und 10, vorzugsweise zwischen 0,5 und 6, beträgt.

**19.** Zusammensetzung nach Anspruch 18,
dadurch gekennzeichnet,
daß die Verbindung der Formel 2 ein Molekulargewicht zwischen 1000 und 4500 hat, wobei das Gewichtsverhältnis der Verbindungen ungefähr 1 beträgt.

**20.** Verfahren zur Desinfektion von Flüssigkeiten,
dadurch gekennzeichnet,
daß man zu diesen Flüssigkeiten mindestens eine Zusammensetzung nach irgendeinem der Ansprüche 7 bis 19 zugibt.

**21.** Verfahren zur Desinfektion von infektiös verunreinigten Oberflächen,
dadurch gekennzeichnet,
daß man diese Oberflächen mit mindestens einer desinfizierenden Zusammensetzung nach irgendeinem der Ansprüche 7 bis 19 in Kontakt bringt.

**22.** Verfahren zur Konservierung von Getränken, in welchem man zu den Getränken eine Zusammensetzung nach irgendeinem der Ansprüche 7 bis 19 zugibt.

**23.** Verfahren zur Konservierung von Lebensmitteln, in welchem man in die Lebensmittel eine Zusammensetzung nach irgendeinem der Ansprüche 7 bis 19 einspritzt.

**24.** Verfahren zur Konservierung von Lebensmitteln, in welchem man die Lebensmittel in eine Zusammensetzung nach irgendeinem der Ansprüche 7 bis 19 eintaucht.

**25.** Mit einer Zusammensetzung nach irgendeinem der Ansprüche 7 bis 19 behandeltes Trägermaterial.

**26.** Fungizide und/oder bakterizide Zusammensetzung,
dadurch gekennzeichnet,
daß sie mindestens eine Verbindung nach irgendeinem der Ansprüche 1 bis 6 enthält.